(19) Europäisches Patentamt
European Patent Office
Office européen des brevets

(11) Publication number: **0 089 901**
**B1**

(12) # EUROPEAN PATENT SPECIFICATION

(45) Date of publication of patent specification: **13.05.87**

(51) Int. Cl.⁴: **C 07 D 207/27,** A 61 K 31/40

(21) Application number: **83400608.2**

(22) Date of filing: **23.03.83**

(54) ***New 2-oxo-1-pyrrolidineacetic acid esters, their methods of production and therapeutic compositions containing the same.***

(30) Priority: **24.03.82 ES 510761**
**24.03.82 ES 510762**

(43) Date of publication of application:
**28.09.83 Bulletin 83/39**

(45) Publication of the grant of the patent:
**13.05.87 Bulletin 87/20**

(84) Designated Contracting States:
**AT BE CH DE FR GB IT LI LU NL SE**

(58) References cited:
**DE-A- 871 154**

**CHEMICAL ABSTRACTS, vol. 87, 1977, page 576, no. 152011p, Columbus, Ohio, USA**

**The file contains technical information submitted after the application was filed and not included in this specification**

(73) Proprietor: **PRODES S.A.**
**rue Trabajo s/n.**
**San Justo Desvern (Barcelona) (ES)**

(72) Inventor: **Sallent, Juan Pi**
**Escolas 30-32**
**La Llagosta (Barcelona) (ES)**

(74) Representative: **Chauchard, Robert et al**
**c/o Cabinet Malémont 42, avenue du Président Wilson**
**F-75116 Paris (FR)**

**EP 0 089 901 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to esters of the formula (I)

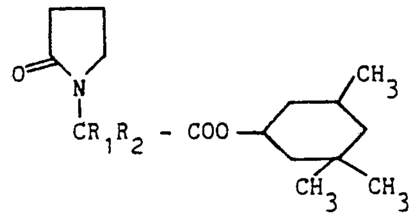

(I)

wherein $R_1$ and $R_2$ are hydrogen, methyl or ethyl.

The esters of the present invention may be produced:

I. By reacting an alkyl 2-oxo-1-pyrrolidineacetate of formula (II)

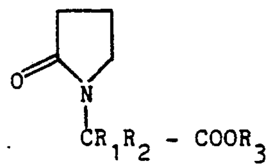

(II)

(wherein $R_1$ and $R_2$ have the same meaning as above, and $R_3$ may be methyl, ethyl, 1-propyl or isopropyl) with 3,3,5-trimethylcyclohexanol (III), in the presence of an alkoxide (usually alkaline) or (III) at 100—180°C (usually 120—160°C) for a period of 6 to 15 hours.

The alkoxide 3,3,5-trimethylcyclohexanol (III) may be prepared "in situ" by previous reaction of the alcohol with the metal or the corresponding hydride under anhydrous conditions.

II. By reacting 2-oxo-pyrrolidine with an alkaline metal or metal hydride (usually sodium) and the resulting alkaline metal derivative is treated with a 3,3,5-trimethylcyclohexyl 2-haloacetate of general formula (IV)

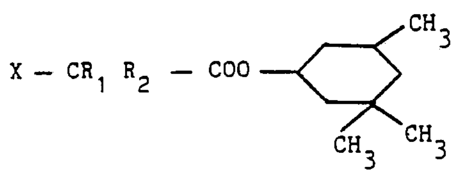

(IV)

in which $R_1$ and $R_2$ have the same meanings as above, and X represents an halogen group (usually chloro or bromo).

Compounds of formula (I) exist as cis/trans isomers by using the appropriate 3,3,5-trimethylcyclohexanol (method I) or 3,3,5-trimethylcyclohexyl haloacetate (method II) the desired isomer will be obtained.

The claimed 2-oxo-1-pyrrolidineacetic acid esters according to this invention are compounds that show pharmacological actions useful for therapeutical purposes.

The following results were obtained in experimental studies:

Electromagnetic Blood Flow Measurements

As can be seen in Meth and Find. Exptl. Clin. Pharmacol. 3 (6) 397—401 (1981)- J. Roca, M. Grau and J. Balasch., the measurement of cerebral blood flow was performed by attaching an electro-magnetic blood flow transducer to the vertebral artery of the anesthetized dogs.

After orotraqueal intubation, artificial respiration with normal air was instituted using a Manley Pulmovent respirator. The correct ventilation of the dogs was checked after all the surgical procedures by determining the $pO_2$, $pCO_2$, and pH in arterial blood samples, as well as several times during the experiment by means of an Astrup apparatus. Blood pressure was measured in left saphenous artery by means of a Bell & Howell pressure transducer, and the heart rate by means of a Beckman ECG device. Recordings were made on an ink-writing Beckman polygraph. Left saphenous vein and right saphenous artery were cannulated with polyethylene catheters for drug administration and blood collection respectively.

Vertebral blood flow was measured by non-cannulating electro-magnetic flowmeter (Carolina Medical Electronics) placed in the vertebral artery.

The Carolina digital flowmeter was connected to an Omniscribe Houston Instruments plotter.

Piracetam and nicotine acid were used as control drugs.

The results are shown in the following table:

TABLE I

| Control and Synthetized Drugs | Effect | Dose |
|---|---|---|
| Piracetam* | No effect | |
| Papaverine | Active | 0.5 mg/kg |
| Nicotinic acid | No effect | — |
| cis/trans-3,3,5-trimethylcyclohexyl 2-oxo-1-pyrrolidineacetate | Active | 1 mg/kg |
| cis-3,3,5-trimethylcyclohexyl 2-(2-oxo-1-pyrrolidinyl)butyrate | Active | 0.5 mg/kg |

* 2-oxo-1-pyrrolidineacetamide

Inespecific protection against letality by "Nootropic" drugs

It is known that the nootropic drugs, as Piracetam, as well as the phenobarbital or hydantoine have a protection on the survival time of animals. When the subjects are submitted to several types of hypoxia, the letality is significantly diminished by the "protection" given by the nootropic drug.

In the intention to increase the accuracy, we have a three trials panel in order to study the effect of the new synthetized drugs.

We have chosen the *hypoxia-hypobara*: the decompression kills the animals, because of the lack of $O_2$. The survival time is clearly increased by the active drugs.

The second trial is quick decapitation of mouse. The head has a typical mandibular movement, known as *gasping*, as searching the breathing air. The gasping time is well prolonged by the active drugs.

The third trial is by *CNK*. Obviously CNK kills the animals. Again the "protected" animals live a good number of seconds more than the controls.

The summary of results is found in Table II.

TABLE II

| Control and Synthetized Drugs | Dose | Hypoxia Hypobara | Anoxia by Gasping | CNK |
|---|---|---|---|---|
| Pentobarbital | 40 mg/kg | Active | Active | Active |
| Difenilhydantoine | 100 mg/kg | Active | Very active | Active |
| Piracetam* | 100 mg/kg | No effect | No effect | No effect |
| cis/trans-3,3,5-trimethylcyclohexyl-2-oxo-1-pyrrolidineacetate | 500 mg/kg | Active | Very active | Very active |
| cis 3,3,5-trimethylcyclohexyl 2(2-oxo-1-pyrrolidinyl)butyrate | 500 mg/kg | Active | Very active | Very active |

* 2-oxo-1-pyrrolidineacetamide

$LD_{50}$ of representative compounds included in Table III.

TABLE III

| Compound | $LD_{50}$ |
|---|---|
| cis/trans-3,3,5-trimethylcyclohexyl 2-oxo-1-pyrrolidineacetate | 3,168 mg/kg |
| cis-3,3,5-trimethylcyclohexyl 2-(2-oxo-1-pyrrolidinyl)butyrate | 2,850 mg/kg |

In view of the above pharmacological and toxicological results, it appears that the compounds of formula (I) may be used as drugs in therapeutics, especially in the treatment of the Raynaud's disease, the arteriosclerosis obliterans, the thromboangiitis obliterans and the senility diseases.

The present invention extends therefore to pharmaceutical compositions containing (a) as active ingredients at least one of the compounds of formula (I) and (b) a pharmaceutically acceptable carrier, these compositions being preferably formulated with a view to their oral administration. Thus they may be administered orally for example in the form of pills, capsules or tablets, in an amount of 50 to 300 mg of active ingredient (1 to 4 times a day).

The following examples will further illustrate the invention.

Example 1

cis/trans-3,3,5-trimethylcyclohexyl 2-oxo-1-pyrrolidineacetate

0,080 gr (0,003 mol) of sodium hydride is added to 4,120 gr (0,029 mol) of cis/trans 3,3,5-trimethylcyclohexanol and the mixture is heated at 70°C until gas evolution ceases. Then 5 gr (0,029 mol) of ethyl 2-oxo-1-pyrrolidineacetate is added and the temperature is raised to 160°C and kept for 12 hours, while distilling ethanol simultaneously.

After reaction, the mixture is fractionally distilled to yield 4,520 gr (57%) of cis/trans 3,3,5-trimethylcyclohexyl 2-oxo-1-pyrrolidineacetate b.p. 150—2°C/0,4 mm.

$R_f$ = 0,33, TLC on silicagel Merck-60 (0,25 mm) eluted with ethyl acetate. Detected with Iodine (esters) and the mixture vainilline 0,5 gr/acetic 30 ml/$H_2SO_4$ (c) 1 ml, at 120°C (cyclohexyl derivatives).

I.R: $(CCl_4)\nu_{max}$ 2950, 1745, 1700, 1490, 1460, 1430, 1420, 1400, 1385, 1360, 1320, 1285, 1200, 1150, 1070, 985, 960 and 940 $cm^{-1}$.

Example 2

cis 3,3,5-trimethylcyclohexyl 2-(2-oxo-1-pyrrolidinyl)butyrate

0,240 gr (0,005 mol) of sodium hydride (50% in mineral oil) is added to 7,100 gr (0,050 mol) of cis 3,3,5-trimethylcyclohexanol and the mixture is heated at 70°C until gas evolution ceases. Then 10 gr (0,050 mol) of 2-(2-oxo-1-pyrrolidinyl)butyrate is added and the temperature is raised to 160°C and kept for 12 hours, while distilling ethanol. After reaction, the mixture is fractionally distilled to yield 9,740 gr (66%) of cis-3,3,5-trimethylcyclohexyl 2-(2-oxo-1-pyrrolidinyl)butyrate. b.p. 153—4°C/0,6 mm.

$R_f$ = 0,41 (TLC under the same conditions as example 1)

I.R: $(CCl_4)\nu_{max}$ 2960, 1740, 1700, 1460, 1385, 1360, 1280 (d), 1190, 1075 and 975 $cm^{-1}$.

Example 3

cis-3,3,5-trimethylcyclohexyl 2-(2-oxo-1-pyrrolidinyl)butyrate

0,240 gr (0,005 mol) of sodium hydride (50% in mineral oil) is added to 7,100 gr (0,050 mol) of cis 3,3,5-trimethylcyclohexanol and the mixture is heated at 70°C until gas evolution ceases. Then 10 gr (0,050 mol) of ethyl 2-(2-oxo-1-pyrrolidinyl)butyrate is added and the temperature is raised to 130°C and kept for 6 hours under 20 mm pressure.

After reaction, the mixture is fractionally distilled to yield 11,658 gr (79%) of cis-3,3,5-trimethylcyclohexyl 2-(2-oxo-1-pyrrolidinyl)butyrate.

Example 4

cis/trans-3,3,5-trimethylcyclohexyl 2-oxo-1-pyrrolidineacetate

A mixture containing 6,120 gr (0,272 mol) of sodium and 275 ml of dry toluene is heated to 97°C while stirring. When sodium is melted, 23,800 gr (0,280 mol) of 2-oxopyrrolidine is added in 45 min., the temperature raises to 107°C while forming the white sodium derivative suspension.

After addition, the mixture is externally heated at 100°C for 2,5 hours until sodium is completely dissolved. Then it is cooled to 70°C and 59,570 gr (0,272 mol) of cis/trans 3,3,5-trimethylcyclohexyl chloroacetate is added in 45 min., and kept for 4 hours. The sodium chloride is filtered and the toluene is removed under reduced pressure.

The residual oil is dissolved in chloroform, washed three times with water and dried over anhydrous sodium sulfate.

Finally the solution is concentrated and the residue fractionally distilled to yield 59,028 gr (81%) of cis/trans-3,3,5-trimethylcyclohexyl 2-oxo-1-pyrrolidineacetate; b.p. 177/8°C/2 mm.

Example 5

cis 3,3,5-trimethylcyclohexyl 2-(2-oxo-1-pyrrolidinyl)butyrate

A mixture containing 5,400 gr (0,234 mol) of sodium and 250 ml of dry toluene is heated to 97°C while stirring. When sodium is melted, 20,485 gr (0,240 mol) of 2-oxopyrrolidine is added dropwise in 20 minutes,

the temperature raises to 107°C while forming the white sodium derivative suspension. After addition, the mixture is externally heated at 100°C for 2,5 hours. Then 57,681 gr (0,234 mol) of cis-3,3,5-trimethylcyclohexyl 2-chlorobutyrate is added and heated at 100°C for 7 hours.

After reaction 250 mol of toluene is added, and the solution is washed twice with 150 ml of water and dried over anhydrous sodium sulfate. Finally the toluene is removed under reduced pressure and the residue is fractionally distilled to yield 53,272 gr (77%) of cis 3,3,5-trimethylcyclohexyl 2-(2-oxo-1-pyrrolidinyl)butyrate

Example 6

When compound of this invention is intended for therapeutic use, types of suitable preparations can be exemplified as follows:

1. Tablet

| | |
|---|---|
| (1) cis/trans-3,3,5--trimethylcyclohexyl 2-oxo-1-pyrrolidineacetate | 5 g |
| (2) Lactose | 75 g |
| (3) Corn starch | 49 g |
| (4) Magnesium stearate | 1 g |
| | 1000 tablets 130 g |

Components (1) and (2) and 29 g of the corn-starch (3) were granulated together with a paste prepared from 11 g of the corn-starch. To these granules were added the remaining 9 g. of corn-starch and component (4).The mixture was then compressed by a tabletting machine to prepare 1000 tablets of 7 mm diameter, each containing 5 mg. of (1).

1. Capsule

| | |
|---|---|
| (1) cis 3,3,5-trimethylcyclohexyl 2-(2-oxo-1-pyrrolidinyl)butyrate | 10 g |
| (2) Lactose | 140 g |
| (3) Cellulose fine powder | 45 g |
| (4) Magnesium stearate | 5 g |
| | 1000 capsules 200 g |

All the materials were mixed and filled into 1000 capsules to prepare capsules each containing 10 mg of (1).

**Claims for the Contracting States: BE CH DE FR GB IT LI LU NL SE**

1. 3,3,5-trimethylcyclohexyl 2-oxo-1-pyrrolidineacetates of the formula (I)

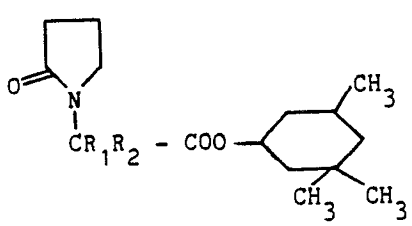

(I)

wherein $R_1$ and $R_2$ are hydrogen, methyl or ethyl, these compounds being in the forms cis, trans or cis/trans.

2. 3,3,5-trimethylcyclohexyl 2-oxo-1-pyrrolidineacetate.
3. 3,3,5-trimethylcyclohexyl 2-(2-oxo-1-pyrrolidinyl)propionate.
4. 3,3,5-trimethylcyclohexyl 2-(2-oxo-1-pyrrolidinyl)butyrate.
5. 3,3,5-trimethylcyclohexyl 2-methyl-2-(2-oxo-1-pyrrolidinyl)propionate.
6. 3,3,5-trimethylcyclohexyl 2-methyl-2-(2-oxo-1-pyrrolidinyl)butyrate.
7. 3,3,5-trimethylcyclohexyl 2-ethyl-2-(2-oxo-1-pyrrolidinyl)butyrate.

8. A pharmaceutical composition which comprises a) as an active ingredient, an effective amount of 2-oxo-1-pyrrolidineacetates as defined in claim 1.- and b) a pharmaceutically acceptable carrier therefore.

9. A method for the production of the 3,3,5-trimethylcyclohexyl 2-oxo-1-pyrrolidineacetates according to claim 1.-, which comprises reacting 2-oxo-1-pyrrolidineacetate of formula (II)

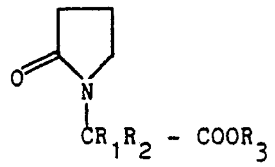

(II)

wherein $R_1$ and $R_2$ are defined as in claim 1, and $R_3$ is methyl, ethyl, l-propyl or isopropyl, with 3,3,5-trimethylcyclohexanol (III) in the presence of its alkoxide (usually alkaline) as catalyst.

10. A process for the production of 3,3,5-trimethylcyclohexyl 2-oxo-1-pyrrolidinacetates according to claim 1, which comprises reacting 2-oxopyrrolidine with an alkali metal or metal hydride and the resulting alkali metal derivative is treated with a 3,3,5-trimethylcyclohexyl 2-haloacetate of the general formula (IV)

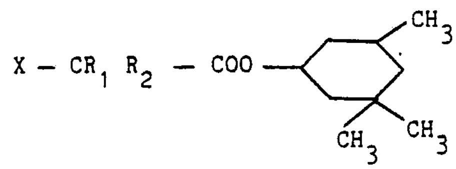

(IV)

in which $R_1$ and $R_2$ have the same meanings as above, and X represents an halogen atom.

**Claims for the Contracting State: AT**

1. A method for the production of 3,3,5-trimethylcyclohexyl 2-oxo-1-pyrrolidineacetates of the formula (I)

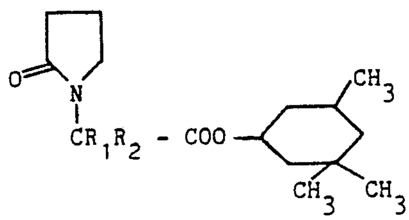

(I)

wherein $R_1$ and $R_2$ are hydrogen, methyl or ethyl, these compounds being in the forms cis, trans or cis/trans, characterized in that it comprises reacting a 2-oxo-1-pyrrolidineacetate of formula (II)

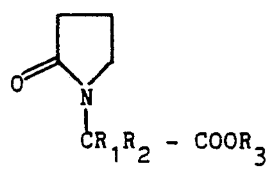

(II)

wherein $R_1$ and $R_2$ have the same meaning as above and $R_3$ is methyl, ethyl, l-propyl or isopropyl, with 3,3,5-trimethylcyclohexanol (III) in the presence of its alkoxide as catalyst.

2. Method according to claim 1, wherein the alkoxide is an alkoxide of an alkaline metal.

3. A method for the production of 3,3,5-trimethylcyclohexyl 2-oxo-1-pyrrolidineacetates of formula (I)

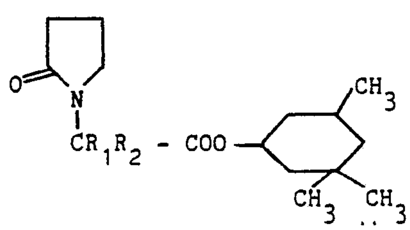

(I)

wherein $R_1$ and $R_2$ are hydrogen, methyl or ethyl, these compounds being in the forms cis, trans or cis/trans, characterized in that it comprises reacting a 2-oxopyrrolidine with an alkaline metal or metal hydride and treating the resulting alkaline metal derivative with 3,3,5-trimethylcyclohexyl 2-haloacetate of formula (IV).

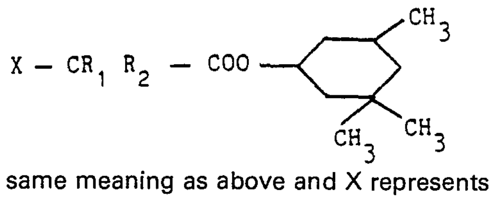 (IV)

wherein $R_1$ and $R_2$ have the same meaning as above and X represents an halogen atom.

**Patentansprüche für die Vertragsstaaten: BE CH DE FR GB IT LI LU NL SE**

1. 3,3,5-Trimethylcyclohexyl-2-oxo-1-pyrrolidinacetate der Formel (I)

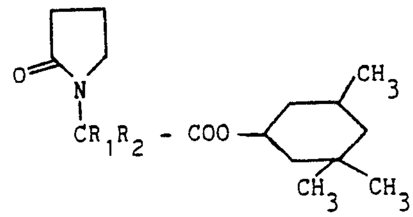 (I)

worin $R_1$ und $R_2$ Wasserstoff, Methyl oder Ethyl bedeuten, wobei diese Verbindungen in den Formen cis, trans oder cis/trans vorliegen.

2. 3,3,5-Trimethylcyclohexyl-2-oxo-1-pyrrolidinacetat.

3. 3,3,5-Trimethylcyclohexyl-2-(2-oxo-1-pyrrolidinyl)propionat.

4. 3,3,5-Trimethylcyclohexyl-2-(2-oxo-1-pyrrolidinyl)butyrat.

5. 3,3,5-Trimethylcyclohexyl-2-methyl-2-(2-oxo-1-pyrrolidinyl)propionat.

6. 3,3,5-Trimethylcyclohexyl-2-methyl-2-(2-oxo-1-pyrrolidinyl)butyrat.

7. 3,3,5-Trimethylcyclohexyl-2-ethyl-2-(2-oxo-1-pyrrolidinyl)butyrat.

8. Pharmazeutische Zusammensetzung, umfassend a) als Wirkstoff eine wirksame Menge der 2-Oxo-1-pyrrolidinacetate nach Anspruch 1 und b) einen pharmazeutisch annehmbaren Träger hierfür.

9. Verfahren zur Herstellung der 3,3,5-Trimethylcyclohexyl-2-oxo-1-pyrrolidinacetate nach Anspruch 1, umfassend das Umsetzen eines 2-Oxo-1-pyrrolidinin acetats der Formel (II)

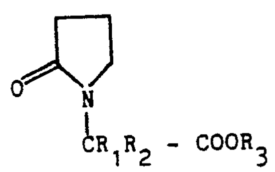 (II)

worin $R_1$ und $R_2$ wie in Anspruch definiert sind und $R_3$ Methyl, Ethyl, I-Propyl oder Isopropyl bedeutet, mit 3,3,5-Trimethylcyclohexanol (III) in Gegenwart dessen Alkoxids (gewöhnlicherweise Alkali) als Katalysator.

10. Verfahren zur Herstellung der 3,3,5-Trimethylcyclohexyl-2-oxo-1-pyrrolidinacetate nach Anspruch 1, umfassend das Umsetzen von 2-Oxopyrrolidin mit einem Alkalimetall oder Metallhydrid und resultilerenden Alkalimetall-Derivates mit einem 3,3,5-Trimethylcyclohexyl-2-halogenacetat der allgemeinen Formel IV

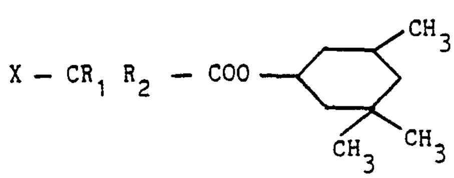 (IV)

worin $R_1$ und $R_2$ die obigen Bedeutungen haben und X ein Halogenatom darstellt.

**Patentansprüche für den Vertragsstaat: AT**

1. Verfahren zur Herstellung von 3,3,5-Trimethylcyclohexyl-2-oxo-1-pyrrolidinacetaten der Formel (I)

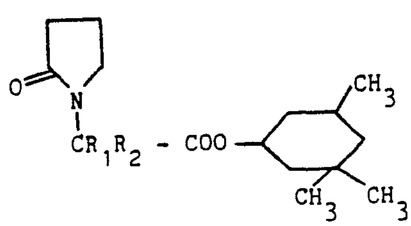 (I)

worin $R_1$ und $R_2$ Wasserstoff, Methyl oder Ethyl bedeuten, wobei diese Verbindungen in den Formen cis, trans oder cis/trans vorliegen dadurch gekennzeichnet, daß es die Umsetzun eins 2-Oxo-1-pyrrolidinacetats der Formel (II)

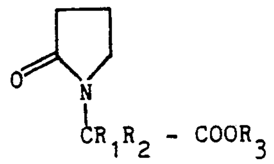

(II)

worin $R_1$ und $R_2$ die oben angegebene Bedeutungen haben und $R_3$ Methyl, Ethyl, I-Propyl oder Isopropyl bedeutet, mit 3,3,5-Trimethylcyclohexanol (III) in Gegenwart dessen Alkoxids als Katalysator umfaßt.

2. Verfahren nach Anspruch 1, wobei das Alkoxid ein Alkoxid eines Alkalimetalls ist.

3. Verfahren zur Herstellung von 3,3,5-Trimethylcyclohexyl-2-oxo-1-pyrrolidinacetaten der Formel (I)

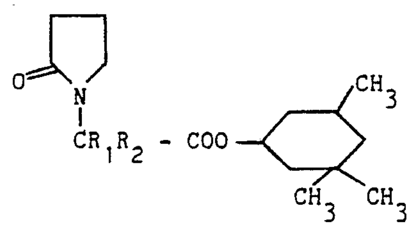

(I)

worin $R_1$ und $R_2$ Wasserstoff, Methyl oder Ethyl bedeuten, wobei diese Verbindungen in den Formen cis, trans oder cis/trans vorliegen dadurch gekennzeichnet, daß es die Umsetzung von 2-Oxopyrrolidin mit einem Alkalimetall oder Metallhydrid und Behandlung des resultierenden Alkalimetall-Derivates mit einem 3,3,5-Trimethylcyclohexyl-2-halogenacetat der Formel (IV) umfaßt.

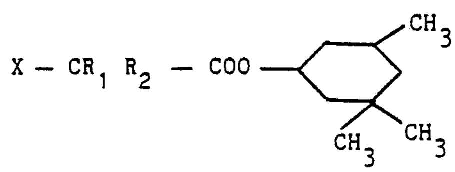

(IV)

worin $R_1$ und $R_2$ die oben angegebenen Bedeutungen haben und X ein Halogenatom darstellt.

**Revendications pour Etats contractants: BE CH DE FR GB IT LI LU NL SE**

1. 3,3,5-triméthylcyclohexyl 2-oxo-1-pyrrolidine acétates de formule (I)

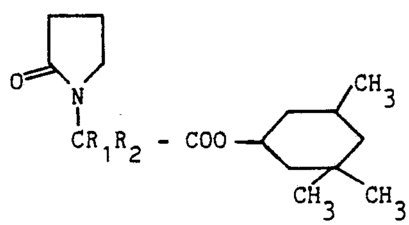

(I)

où $R_1$ et $R_2$ sont l'hydrogène, méthyle ou éthyle, ces composés étant sous la forme cis, trans ou cis/trans.

2. 3,3,5-triméthylcyclohexyl 2-oxo-1-pyrrolidine acétate.
3. 3,3,5-triméthylcyclohexyl 2-(2-oxo-1-pyrrolidinyl)propionate.
4. 3,3,5-triméthylcyclohexyl 2-(2-oxo-1-pyrrolidinyl)butyrate.
5. 3,3,5-triméthylcyclohexyl 2-méthyl-2-(2-oxo-1-pyrrolidinyl)propionate.
6. 3,3,5-triméthylcyclohexyl 2-méthyl-2-(2-oxo-1-pyrrolidinyl)butyrate.
7. 3,3,5-triméthylcyclohexyl 2-éthyl-2-(2-oxo-1-pyrrolidinyl)butyrate.
8. Composition pharmaceutique qui comprend a) à titre de principe actif, une quantité efficace des 2-oxo-1-pyrrolidine acétates définis à la revendication 1 et b) un support pharmaceutiquement acceptable.
9. Procédé de préparation des 3,3,5-triméthylcyclohexyl -2-oxo-1-pyrrolidine acétates selon la revendication 1, comprenant la réaction d'un 2-oxo-1-pyrrolidine acétate de formule (II):

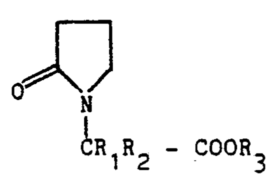

(II)

où $R_1$ et $R_2$ sont tels que définis à la revendication 1 et $R_3$ est méthyle, éthyle, I-propyle ou isopropyle, avec le 3,3,5-triméthylcyclohexanol (III) en présence de son alcoolate (habituellement alcalin) à titre de catalyse.

10. Procédé de préparation des 3,3,5-triméthylcyclohexyl 2-oxo-1-pyrrolidine acétates selon la revendication 1, comprenant la réaction de la 2-oxopyrrolidine avec un métal alcalin ou un hydrure métallique, le dérivé de métal alcalin résultant étant traité avec un 2-haloacétate de 3,3,5-trimèthylcyclohexyle de formule générale (IV):

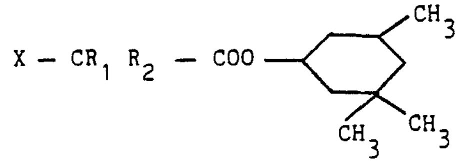

(IV)

où $R_1$ et $R_2$ ont les mêmes significations que ci-dessus et X représente un atome d'halogène.

**Revendications pour l'Etat contractant: AT**

1. Procédé de préparation des 3,3,5-triméthylcyclohexyl 2-oxo-1-pyrrolidine acétates de formule (I):

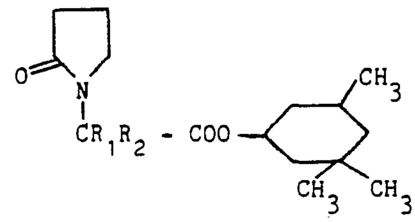

(I)

dans laquelle $R_1$ et $R_2$ sont hydrogène, méthyle ou éthyle, ces composés étant sous la forme cis, trans ou cis/trans, caractérisé en ce qu'il comprend la réaction d'un 2-oxo-pyrrolidine acétate de formule (II):

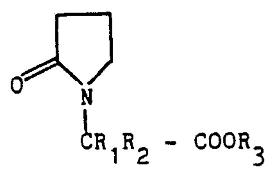

(II)

où $R_1$ et $R_2$ ont la même signification que ci-dessus et $R_3$ représente méthyle, éthyle, 1-propyle ou isopropyle, avec le 3,3,5-triméthylcyclohexanol (III) en présence de son alcoolate à titre de catalyseur.

2. Procédé selon la revendication 1, dans lequel l'alcoolate est un alcoolate d'un métal alcalin.

3. Procédé pour la préparation des 3,3,5-triméthylcyclohexyl 2-oxo-1-pyrrolidine acétate de formule (I):

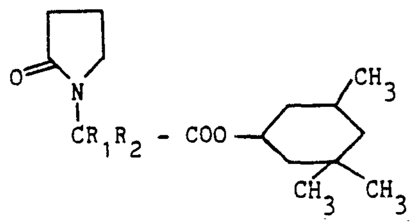

(I)

où $R_1$ et $R_2$ sont hydrogène méthyle ou éthyle, ces composés étant sous la forme cis, trans ou cis/trans, caractérisé en ce qu'il comprend la réaction de la 2-oxo pyrrolidine avec un métal alcalin ou un hydrure métallique et le traitement du dérivé de métal alcalin résultant avec un 2-haloacétate de 3,3,5-triméthylcyclohexyle de formule (IV):

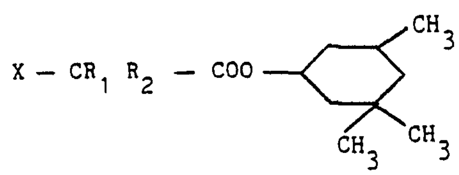

(IV)

où $R_1$ et $R_2$ ont les mêmes significations que ci-dessus et X représente un atome d'halogène.